## Europäisches Patentamt
## European Patent Office
## Office européen des brevets

(11) Veröffentlichungsnummer: **0 368 067**

**A1**

# (12) EUROPÄISCHE PATENTANMELDUNG

(21) Anmeldenummer: **89119600.8**

(22) Anmeldetag: **23.10.89**

(51) Int. Cl.5: **A61B 6/00**

(30) Priorität: **09.11.88 DE 8814203 U**

(43) Veröffentlichungstag der Anmeldung:
**16.05.90 Patentblatt 90/20**

(84) Benannte Vertragsstaaten:
**AT BE CH DE ES FR GB GR IT LI LU NL SE**

(71) Anmelder: **Muthmann, Karl-Dieter**
**Siegfriedstrasse 5**
**D-5810 Witten(DE)**

(72) Erfinder: **Muthmann, Karl-Dieter**
**Siegfriedstrasse 5**
**D-5810 Witten(DE)**

(74) Vertreter: **Patentanwälte Wenzel & Kalkoff**
**Flasskuhle 6 Postfach 2448**
**D-5810 Witten(DE)**

(54) **Röntgenuntersuchungseinrichtung.**

(57) Eine Röntgenuntersuchungseinrichtung weist eine Säule (1) auf, an der eine Halterung (3) mit einer Laufrastereinrichtung (4) und einer Röntgenröhre (8) vertikal verfahrbar ist. Zur Erreichung einer Vielzahl von Aufnahmemöglichkeiten am liegenden, stehenden oder sitzenden Patienten aus verschiedenen Richtungen ist die Halterung (3) auch horizontal verfahrbar, die Laufrastereinrichtung (4) am seitlichen Ende der Halterung (3) um eine vertikale Achse (Av) schwenkbar, die Röntgenröhre an einem Ende eines im wesentlichen C-förmigen Tragarmes (6) gehalten, dessen anderes Ende (5) an der Laufrastereinrichtung (4) angeordnet und so befestigt ist, daß die Röntgenröhre (8) gemeinsam mit der Laufrastereinrichtung (4) um die vertikale Achse (Av) schwenkbar ist und die Laufrastereinrichtung (4) zusätzlich um eine horizontale Achse (Ah) drehbar ist.

EP 0 368 067 A1

## Röntgenuntersuchungseinrichtung

Die Erfindung betrifft eine Röntgenuntersuchungseinrichtung mit einer Säule, an der eine Halterung mit einer Laufrastereinrichtung und einer Röntgenröhre vertikal verfahrbar ist.

Insbesondere für Aufnahmen und Durchleuchtungen am liegenden Patienten ist es in der Unfallmedizin wichtig, daß Röntgenuntersuchungen zur Verfügung stehen, die Aufnahmen und Durchleuchtungen des auf dem Patientenlagerungstisch liegenden Patienten zulassen, ohne daß der Körper des möglicherweise bewußtlosen Patienten für Aufnahmen und Durchleuchtungen in eine andere Position gebracht werden muß. Es kommt also darauf an, daß die Laufrastereinrichtung ebenso wie die Röntgenröhre gegenüber dem Patienten leicht für eine Vielzahl verschiedener Aufnahmen aus verschiedenen Richtungen gegenüber dem Patienten anzuordnen sind. Bisher ist der Aufwand für in dieser Weise vielseitige Röntgenuntersuchungseinrichtungen beträchtlich. Deshalb besteht Bedarf für eine solche vielseitige Einrichtung mit einfachem Aufbau, die mit, gemessen an ihrer Vielseitigkeit, geringem Aufwand herstellbar und relativ leicht bedienbar ist.

Hierfür ist nach der Erfindung die Anspruch 1 entnehmbare Röntgenuntersuchungseinrichtung vorgesehen.

Aufgrund der horizontalen sowie vertikalen Verfahrbarkeit der in entsprechenden Führungen an der Säule gelagerten Halterung sowie vor allem wegen der Verschwenkbarkeit der Laufrastereinrichtung und der über den etwa C-förmigen Tragrahmen in entsprechender Anordnung von der Laufrastereinrichtung gehaltenen Röntgenröhre sowie aufgrund der Drehbarkeit sowohl der Laufrastereinrichtung als auch des Tragarms mit der Röntgenröhre um eine horizontale Achse gegenüber der Halterung wird eine bisher nicht erreichte Vielseitigkeit der möglichen Anordnung von Röntgenröhre und Laufrastereinrichtung gegenüber dem auf einem Patientenlagerungstisch liegenden Patienten erreicht.

Neben den schon bisher mit üblichen Einrichtungen möglichen Aufnahmen und Durchleuchtungen am sitzenden und stehenden Patienten, wobei praktisch jeder Körperteil bei entsprechender Positionierung der Laufrastereinrichtung und der Röntgenröhre für Aufnahmen bzw. Durchleuchtungen in Frage kommt, sind nun vor allem auch seitliche Aufnahmen möglich. Dabei befindet sich die Laufrastereinrichtung in vertikaler Lage neben der betreffenden Stirnseite der Halterung, und die Röntgenröhre ist über den C-förmigen Tragarm in entsprechendem Abstand vor der Laufrastereinrichtung gehalten, so daß ohne weiteres seitliche Aufnahmen am Patienten durchführbar sind, der beispielsweise auf einem entsprechend an die Säule herangefahrenen Patientenlagerungstisch liegt, der sich dann von oben gesehen zwischen der vertikal stehenden Laufrastereinrichtung und der Röntgenröhre befindet.

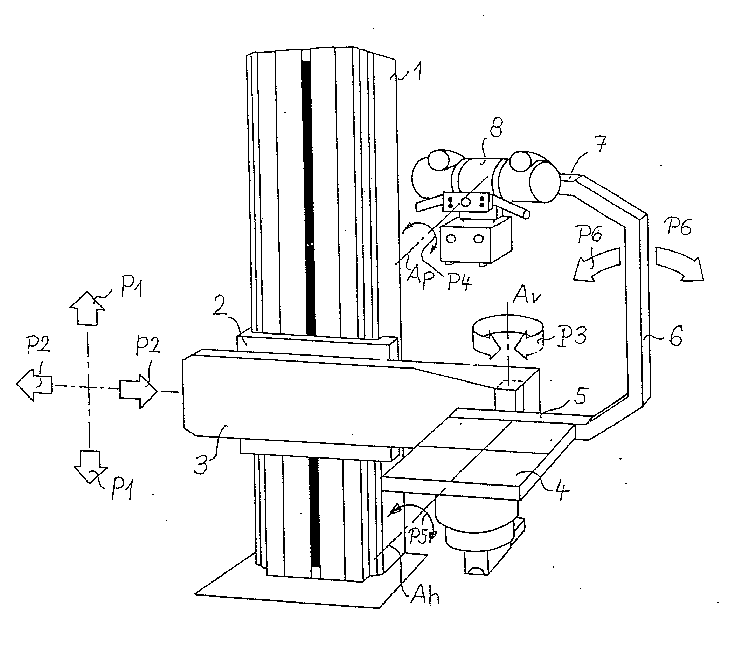

Alle anderen Aufnahmemöglichkeiten ergeben sich aufgrund der erfindungsgemäßen Merkmale in Verbindung mit der benutzten herkömmlichen Technik, vor allem unter Heranziehung der beigefügten Zeichnung, für den Fachmann von selbst.

Vorteilhafte Weiterbildungen der Erfindung sind in den Ansprüchen 2 und 3 angegeben, ohne daß es hierfür einer besonderen Erläuterung bedarf. Die Vorteile liegen für den Fachmann auf der Hand.

Die Zeichnung zeigt in schematischer perspektivischer Ansicht ein Ausführungsbeispiel einer erfindungsgemäßen Röntgenuntersuchungseinrichtung.

An einer im Untersuchungsraum entweder mit einer entsprechenden Bodenbefestigung freistehenden oder an der Wand befestigten Säule 1 ist mittels herkömmlicher Führungen und Antriebe eine Tragplatte 2 vertikal verfahrbar, wie durch die Pfeile P1 angedeutet ist.

An der Tragplatte 2 ist eine Halterung 3 mittels entsprechender zwischen der Tragplatte 2 und der Halterung 3 angeordneter, in der Zeichnung nicht dargestellter Führungen sowie Antriebe horizontal verfahrbar angeordnet, wie durch die Pfeile P2 angedeutet ist.

Am in der Zeichnung rechten sowie seitlichen Ende der Halterung 3 ist eine nicht dargestellte Lagervorrichtung angeordnet, mittels der eine Laufrastereinrichtung 4 in der dargestellten Anordnung um eine vertikale Achse Av schwenkbar ist. Der Schwenkwinkel, angedeutet durch den Doppelpfeil P3, ist mindestens so groß, daß die Laufrastereinrichtung 4 aus der in der Zeichnung dargestellten Lage vor der Halterung 3 in eine nicht dargestellte seitliche Lage neben der Halterung 3 schwenkbar ist, was einem Schwenkwinkel von 90° entspricht.

An der der Halterung 3 zugewandten Seite der Laufrastereinrichtung 4 ist ein Ende 5 eines, wie aus der Zeichnung ersichtlich, etwa C-förmigen Tragarms 6 befestigt. An dem gegenüberliegenden Ende 7 des Tragarms 6 ist eine Röntgenröhre 8 angebracht, die um eine Achse Ap schwenkbar sein kann, wie durch den Doppelpfeil P4 angedeutet ist. Die Achse Ap ist parallel zu einer im folgenden erläuterten horizontalen Achse Ah.

Die Lagerung der Laufrastereinrichtung 4 an der Halterung 3 ist so gestaltet, daß sie nicht nur eine Schwenkbewegung der Laufrastereinrichtung, des Tragarms 6 und der Röntgenröhre 8 um die

2

vertikale Achse Av zuläßt, sondern gleichzeitig sind die vorgenannten Bauteile um eine horizontale Achse Ah drehbar gelagert, unterstützt durch einen entsprechenden elektromotorischen Antrieb, wobei der Drehwinkel durch den Doppelpfeil P5 angedeutet ist.

In dem dargestellten Ausführungsbeispiel ist der Tragarm 6 mit der Röntgenröhre 8 durch die Befestigung an der Laufrastereinrichtung 4 mit dieser gemeinsam als Einheit um die Achse Ah drehbar. Auf diese Weise wird ein einmal eingestellter Abstand sowie die Parallelität zwischen der Laufrastereinrichtung 4 und der Röntgenröhre 8 stets beibehalten.

Es ist jedoch auch möglich, abweichend von dem vorgenannten und dargestellten Ausführungsbeispiel, das Ende 5 des Tragarms 6 unabhängig von der Laufrastereinrichtung 4 um die Achse Ah drehbar anzuordnen, so daß diese relative Drehbeweglichkeit des Tragarms 6 und der Laufrastereinrichtung 4 gestattet, die Röntgenröhre 8 schräg auf die Bildebene der Laufrastereinrichtung 4 zu richten. Diese Drehbeweglichkeit des Arms 6 ist durch die Pfeile P6 angedeutet.

Der Fachmann kann die Vielzahl der Einstellungsmöglichkeiten am stehenden, sitzenden und insbesondere auch am liegenden Patienten ohne weiteres der Zeichnung in Verbindung mit der vorstehenden Beschreibung entnehmen.

Es sei besonders darauf hingewiesen, daß die Verbindung zwischen dem Laufraster 4 und der Röntgenröhre 8 nicht notwendigerweise ein Tragarm in C-Form sein muß. Diese wurde gewählt, um eine leichte Handhabung zu ermöglichen und vor allem den notwendigen Freiraum zwischen der Laufrastereinrichtung 4 und der Röhre 8 zu schaffen. Es kann auch ein Tragarm oder ein anderweitiges Verbindungsglied mit anderer Formgestaltung vorgesehen sein.

Die Laufrastereinrichtung 4 kann in Verbindung mit jeder Kassette verwendet werden, und zwar im Gegensatz zu einem Kassettenkasten, dessen Verwendung auf ein einziges Format beschränkt ist. Außerdem gestattet die gewählte Anordnung, wie die Zeichnung zeigt, die Benutzung der Laufrastereinrichtung 4 in Verbindung mit einem Bildverstärker, in der Praxis derzeit bis zu einem BV-Format von 40 cm (Format des Bildverstärker-Eingangsfeldes).

## Ansprüche

1. Röntgenuntersuchungseinrichtung mit einer Säule, an der eine Halterung mit einer Laufrastereinrichtung und einer Röntgenröhre vertikal verfahrbar ist, **gekennzeichnet** durch folgende Merkmale:

die Halterung (3) ist an der Säule (1) auch horizontal verfahrbar,

die Laufrastereinrichtung (4) ist am seitlichen Ende der Halterung (3) um eine vertikale Achse (Av) derart schwenkbar gelagert, daß sie zumindest zwischen einer vorderen Lage vor der Halterung (3) und einer hiervon um einen Schwenkwinkel von 90° entfernten seitlichen Lage neben der Halterung (3) verschwenkbar ist,

die Röntgenröhre (8) ist an einem Ende (7) eines im wesentlichen C-förmigen Tragarms (6) gehalten, dessen anderes Ende (5) an der Laufrastereinrichtung (4) derart angeordnet ist, daß sich die Röntgenröhre (8) in Aufnahmeposition gegenüber der Laufrastereinrichtung (4) befindet und gemeinsam mit der Laufrastereinrichtung (4) um die vertikale Achse (Av) verschwenkbar ist,

und die Laufrastereinrichtung (4) und das andere Ende (5) das Tragarms (6) sind gegenüber der Halterung (3) um eine horizontale Achse (Ah) drehbar, entweder gemeinsam als Einheit oder einzeln sowie unabhängig voneinander.

2. Röntgenuntersuchungseinrichtung nach Anspruch 1, dadurch **gekennzeichnet**, daß die Röntgenröhre (8) um eine zur horizontalen Achse (Ah) der Drehbewegung der Laufrastereinrichtung (4) und des anderen Endes (5) des Tragarms (6) parallele Achse (Ap) in zwei Richtungen verschwenkbar ist.

3. Röntgeneinrichtung nach Anspruch 1 oder 2, dadurch **gekennzeichnet**, daß die Halterung (3) an der Säule (1) mittels eines Präzisionsantriebes horizontal derart verfahrbar ist, daß Aufnahmen mit digitaler Bildsubtraktion durchführbar sind.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 224 886 (SIEMENS AG) * Figuren 1-3; Seite 3, Zeile 27 - Seite 5, Zeile 34 * | 1 | A 61 B 6/00 |
| A | | 3 | |
| Y | US-A-3 500 045 (ROSSI) * Figuren 1,2; Spalte 3, Zeile 24 - Spalte 4, Zeile 7 * | 1 | |
| A | EP-A-0 168 109 (PHILIPS PATENTVERWALTUNG GmbH) * Figur 1; Seite 3, Zeile 14 - Seite 5, Zeile 7 * | 1,2 | |
| A | US-A-4 653 083 (ROSSI) * Figuren 1,2; Spalte 2, Zeilen 3-29 * | 1 | |
| A | EP-A-0 231 969 (N.V. PHILIPS' GLOEILAMPENFABRIEKEN) * Figur 1; Spalte 4, Zeilen 30-44 * | 3 | |

RECHERCHIERTE SACHGEBIETE (Int. Cl.5)

A 61 B

Der vorliegende Recherchenbericht wurde für alle Patentansprüche erstellt

| Recherchenort | Abschlußdatum der Recherche | Prüfer |
|---|---|---|
| DEN HAAG | 28-12-1989 | CHEN A.H. |